(19) Europäisches Patentamt
European Patent Office
Office européen des brevets

(11) EP 1 119 248 B1

# (12) EUROPÄISCHE PATENTSCHRIFT

(45) Veröffentlichungstag und Bekanntmachung des Hinweises auf die Patenterteilung:
**02.02.2005 Patentblatt 2005/05**

(21) Anmeldenummer: **99950602.5**

(22) Anmeldetag: **02.10.1999**

(51) Int Cl.[7]: **A01N 31/08**, A61K 7/48, A61K 7/06

(86) Internationale Anmeldenummer:
**PCT/EP1999/007313**

(87) Internationale Veröffentlichungsnummer:
**WO 2000/021368 (20.04.2000 Gazette 2000/16)**

## (54) HYDROXYSTILBENVERBINDUNGEN ALS MIKROBIZIDE WIRKSUBSTANZEN

HYDROXYSTILBENE COMPOUNDS USED AS MICROBICIDAL ACTIVE SUBSTANCES

COMPOSES HYDROXYSTILBENE UTILISES COMME PRINCIPES ACTIFS MICROBICIDES

(84) Benannte Vertragsstaaten:
**AT BE CH CY DE DK ES FI FR GB GR IE IT LI LU MC NL PT SE**

(30) Priorität: **09.10.1998 EP 98811006**

(43) Veröffentlichungstag der Anmeldung:
**01.08.2001 Patentblatt 2001/31**

(73) Patentinhaber: **Ciba Specialty Chemicals Holding Inc.**
**4057 Basel (CH)**

(72) Erfinder:
- **HÖLZL, Werner**
  **F-68440 Eschentzwiller (FR)**
- **OCHS, Dietmar**
  **D-79650 Schopfheim (DE)**
- **HAAP, Wolfgang**
  **D-79639 Grenzach-Wyhlen (DE)**
- **PUCHTLER, Karin**
  **D-79592 Fischingen (DE)**
- **SCHNYDER, Marcel**
  **CH-4127 Birsfelden (CH)**

(56) Entgegenhaltungen:
**EP-A- 0 953 345** **WO-A-95/03695**
**FR-A- 2 020 995**

- **CHEMICAL ABSTRACTS, vol. 128, no. 13, 1998 Columbus, Ohio, US; abstract no. 158748, ASHITA, TSUTOMU: "Body cleansers containing Yucca extracts and their manufacture" XP002096223 & JP 10 045566 A**
- **CHEMICAL ABSTRACTS, vol. 128, no. 7, 1998 Columbus, Ohio, US; abstract no. 79811, ASHIDA ,TSUTOMU: "Cosmetics containing Yucca extracts and their manufacture" XP002096224 & JP 09 328410 A**
- **CHEMICAL ABSTRACTS, vol. 108, no. 3, 1988 Columbus, Ohio, US; abstract no. 17670, GIESBRECHT ET AL.: "Antibacteria and antifungal activity of Gnetum compounds" XP002096225 & ACTA AMAZONICA, Bd. 15, Nr. 3-5, 1985, Seiten 321-325,**

Bemerkungen:
Die Akte enthält technische Angaben, die nach dem Eingang der Anmeldung eingereicht wurden und die nicht in dieser Patentschrift enthalten sind.

EP 1 119 248 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft die Verwendung von Hydroxystilbenverbindungen zur Abtötung und/oder Hemmung des Wachstums von pathogenen grampositiven und gramnegativen Bakterien sowie Bakterien der Hautflora, Hefen und Schimmelpilzen.

C.A. 128:15878 (1998) offenbart Hautreinigungsmittel, die Yucca-Extracte enthalten, die aus den Wurzeln oder Stämmen dieser Pflanze erhältlich sind. Die Extrakte enthalten verschiedene Wirksubstanzen, u.a. auch Resveratrol.

C.A. 128:79811 (1998) offenbart Haut-Konditioniermittel, die die Yucca-Extracte enthalten, die aus den Wurzeln oder Stämmen dieser Pflanze erhältlich sind.

C.A. 108:17670 (1988) offenbart, dass Extrakte der Pflanze Gnetum Planiculatum und Gnetum schwackeanum und Substanzen, die aus dieser Pflanze isoliert werden, antibiotische Eigenschaften aufweisen. In dieser Referenz wird erwähnt, dass Resveratrol antimikrobielle Eigenschaften gegenüber Mycobacterium albicans aufweist.

WO 95/03695 offenbart neue fungizide Eigenschaften von gewissen Stifbenderivaten.

FR-A-2,020,995 offenbart die antimikrobielle Verwendung von gewissen Hydroxystilbenverbindungen , insbesondere als Algizid.

Die erfindungsgemäss eingesetzten Hydroxystilbenverbindungen entsprechen der Formel

(1) 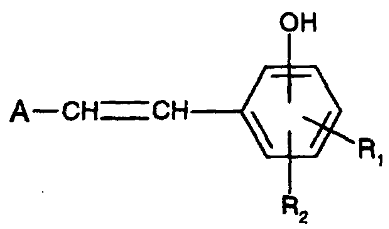 ,

worin

A einen Rest der Formel (1a)

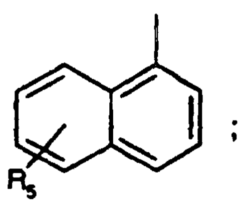 ;

und

$R_1$, $R_2$, $R_3$, $R_4$ und $R_5$, unabhängig voneinander Wasserstoff, Halogen, Hydroxy, $C_1$-$C_{16}$-Alkyl, $C_1$-$C_{16}$-Alkoxy, Phenyl; $C_1$-$C_3$-Phenylalkyl; $C_6$-$C_{10}$-Aryloxy, Amino, Mono-$C_1$-$C_5$-Alkylamino, Di-$C_1$-$C_5$-Alkylamino, oder -$NO_2$;

bedeuten.

$C_1$-$C_{16}$-Alkyl sind geradkettige oder verzweigte Alkylreste wie z.B. Methyl, Ethyl, n-Propyl, isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl oder Hexadecyl.

$C_1$-$C_{16}$-Alkoxy bedeutet z.B. Methoxy, Ethoxy, n-Propoxy, Isopropoxy, n-Butoxy, sek.Butoxy, tert.Butoxy, Amyloxy, Isoamyloxy oder tert.Amyloxy, Hexyloxy, Heptyloxy, Octyloxy, Isooctyloxy, Nonyloxy, Decyloxy, Undecytoxy, Dodecyloxy, Tetradecyloxy, Pentadecyloxy oder Hexadecyloxy.

$C_6$-$C_{10}$-Aryloxy bedeutet Phenoxy oder Naphthyloxy.

Halogen bedeutet Fluor, Chlor, Brom oder lod.

Die erfindungsgemäss verwendeten Hydroxystilbene können als E- oder Z-Isomere vorliegen. Vorzugsweise

liegen sie als E-Isomere vor.

Interessante Verbindungen, die erfindungsgemäss verwendet werden, sind Dihydroxystilbene, d.h. Verbindungen der Formel (1), worin

$R_1$ und $R_2$ Hydroxy

bedeuten.

Weiterhin sind Verbindungen der Formel

(3) 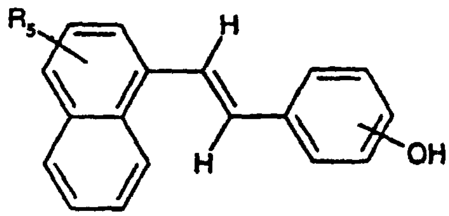

bevorzugt, worin

$R_5$ die in Formel (1) angegebene Bedeutung hat und insbesondere Wasserstoff; bedeutet.

Die Herstellung der Verbindungen der Formel (1 ) erfolgt nach an sich bekannten Verfahren durch Umsetzung eines Alkylphosphits, wie z.B. Triethylphosphit mit einem Benzylhalogenid, vorzugsweise Benzylbromid. Man erhält die Phosphonatzwischenstufe (1. Stufe).

Anschliessend setzt man die Phosphonatzwischenstufe mit einem Alkoxybenzaldehyd um (2. Stufe). Die anschliessende Dealkylierung (3. Stufe) erfolgt nach üblichen Methoden.

Der gesamte Reaktionsablauf lässt sich folgendermassen darstellen:

1. Stufe:

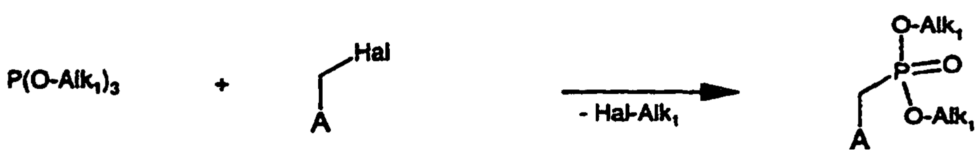


2. Stufe:

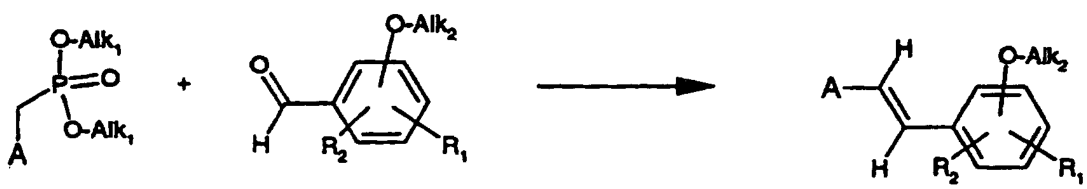

3. Stufe:

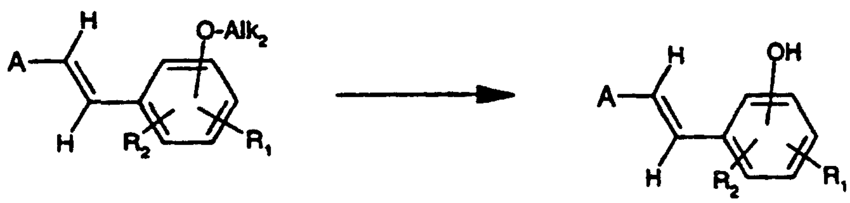

Nähere Einzelheiten für diese Reaktion sind der Can. J. Chem. 48, 1554 (1970) zu entnehmen.

In einer weiteren Variante lassen sich die erfindungsgemässen Hydroxystilbenverbindungen in einer Festphasensynthese unter Verwendung eines Tritylharzes herstellen. Die Herstellung erfolgt nach folgendem Schema:

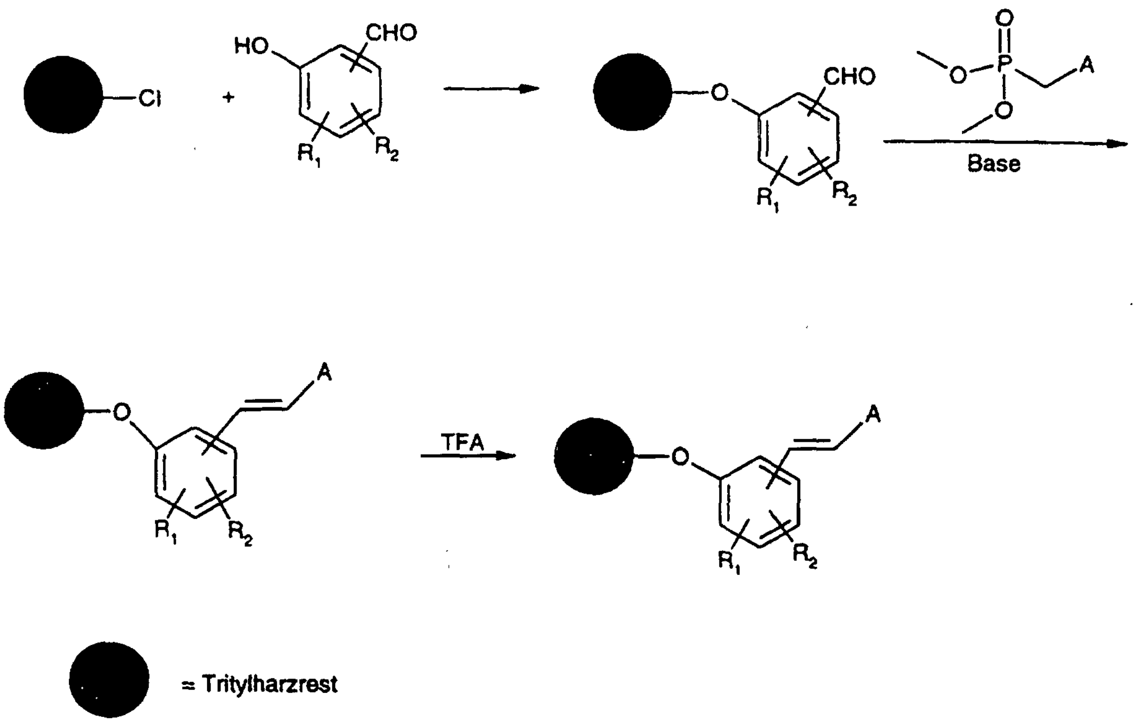


$R_1$, $R_2$ und A haben dabei die in Formel (1) angegebene Bedeutung.

Die Synthesemethode basiert auf der Literaturvorschrift von R. Willard et al., Chemistry & Biology, *2*, 1995, 45-51. Der Unterschied des erfindungsgemässen Herstellungsverfahrens besteht in der Verwendung des Tritylharzes und der unterschiedlichen Methode zur Beladung des Harzes.

Nähere Einzelheiten über das erfindungsgemässe Herstellungsverfahren sind den entsprechenden Beispielen zu entnehmen.

Die erfindungsgemäss eingesetzten Hydroxystilbenverbindungen zeigen ausgeprägte antimikrobielle Wirkung, insbesondere gegen pathogene grampositive und gramnegative Bakterien sowie gegen Bakterien der Hautflora, wie z.B. Corynebacterium xerosis (Bakterien, die Körpergeruch verursachen), ausserdem gegen Hefen und Schimmelpilze. Sie eignen sich daher insbesondere zur Desinfektion der Haut und Schleimhäute sowie Hautanhangsgebilden (Haare), ganz besonders zur Hände- und Wunddesinfektion.

Sie sind daher geeignet als antimikrobielle Wirksubstanzen und Konservierungsmittel in Körperpflegemitteln, wie z.B. Shampoos, Badezusätzen, Haarpflegemitteln, flüssigen und festen Seifen (auf Basis synthetischer Tenside und Salze von gesättigten und/oder ungesättigten Fettsäuren), Lotionen und Cremes, Deodorantien, anderen wässrigen oder alkoholischen Lösungen, z.B. Reinigungslösungen für die Haut, feuchten Reinigungstüchern, Ölen oder Pudern.

Die erfindungsgemäss verwendeten Verbindungen der Formel (1) können in Körperpflegemitteln eingesetzt werden, die neben der Verbindung der Formel (1) kosmetisch verträgliche Träger- oder Hilfsstoff entahlten.

Das Körperpflegemittel enthält 0,01 bis 15, vorzugsweise 0,1 bis 10 Gew.-%, bezogen auf das Gesamtgewicht der Zusammensetzung, der Hydroxystilbenverbindung der Formel (1) und kosmetisch verträgliche Hilfsstoffe.

Je nachdem, in welcher Form das Körperpflegemittel vorliegt, weist es neben der Stilbenverbindung der Formel (1) noch weitere Bestandteile auf, wie z.B. Sequestriermittel, Farbstoffe, Parfümöle, Verdickungs- bzw. Festigungsmittet (Konsistenzregler), Emmollients, UV-Absorber, Hautschutzmittet, Antioxidantien, die mechanischen Eigenschaften verbessernde Additive wie Dicarbonsäuren und/oder Al-, Zn-, Ca-, Mg-Salze von $C_{14}$-$C_{22}$-Fettsäuren und gegebenenfalls Konservierungsmittel.

Das Körperpflegemittel kann als Wasser-in-Öl- oder Öl-in-Wasser-Emulsion, als alkoholische oder alkoholhaltige Formulierung, als vesikulare Dispersion eines ionischen oder nichtionischen amphiphilen Lipids, als Gel, fester Stift oder als AerosolFormulierung formuliert werden.

Als Wasser-in-Öl- oder Öl-in-Wasser-Emulsion enthält der kosmetisch verträgliche Hilfsstoff vorzugsweise 5

bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexytenglycol, Glycerin und Sorbitol.

Kosmetische Formulierungen beinhalten verschiedene kosmetische Mittel. Insbesondere kommen z.B. die folgenden Mittel in Betracht:

- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-Ups in Form von Tages- oder Pudercremes, Gesichtspuder (lose und gepresst), Rouge oder Creme-Make-Ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depitation), wie z.B. Haarentfemungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Eine antimikrobielle Seife hat z.B. folgende Zusammensetzung:

0,01 bis 5 Gew.-% der Verbindung der Formel (1)
0,3 bis 1 Gew.-% Titandioxid,
1 bis 10 Gew.-% Stearinsäure
ad 100% Seifengrundlage, wie z.B. die Natriumsalze der Talgfett- und Kokosfettsäure oder Glycerine.

Ein Schampoo hat z.B. die folgende Zusammensetzung:

0,01 bis 5 Gew.-% der Verbindung der Formel (1),
12,0 Gew.-% Natrium-Laureth-2-sulfat,
4,0 Gew.-% Cocamidopropylbetain,
3,0 Gew.-% NaCl und
Wasser ad 100%.

Ein Deodorant hat z.B. die folgende Zusammensetzung:

0,01 bis 5 Gew.-% der Verbindung der Formel (1),
60 Gew.-% Ethanol,
0,3 Gew.-% Parfümöl, und
Wasser ad 100%.

Die erfindungsgemäss verwendeten Verbindungen der Formel (1) können auch in einer oralen Zusammensetzung, enthaltend 0,01 bis 15 Gew.-%, bezogen auf das Gesamtgewicht der Zusammensetzung, der Verbindung der Formel (1) und oral verträgliche Hilfsstoffe ,verwendet werden.

Beispiel für eine orale Zusammensetzung:

10 Gew.-% Sorbitol,
10 Gew.-% Glycerin,
15 Gew.-% Ethanol,
15 Gew.-% Propylenglykol,
0,5 Gew.-% Natriumlaurylsulfat,
0,25 Gew.-% Natriummethylcocyltaurat,
0,25 Gew.-% Polyoxypropylen/Polyoxyethylen-Blockcopolymer,
0,10 Gew.-% Pfefferminzgeschmacksstoff.
0,1 bis 0,5 Gew.-% einer Verbindung der Formel (1), und
48,6 Gew.-% Wasser.

Die orale Zusammensetzung kann z.B. in Form eines Gels, einer Paste, einer Creme oder einer wässrigen Zubereitung (Mundwasser) vorliegen.

Weiterhin kann die orale Zusammensetzung Verbindungen enthalten, die Fluoridionen freisetzen, die gegen die Bildung von Karies wirksam sind, z.B. anorganische Fluoridsalze, wie z.B. Natrium-, Kalium-, Ammonium- oder Calciumfluorid oder organische Fluoridsalze, wie z.B. Aminfluoride, die unter dem Handelsnamen Olafluor bekannt sind.

Weiterhin eignen sich die erfindungsgemäss verwendeten Stilbenverbindungen der Formel (1) für die Behandlung von textilen Fasermaterialien. Es handelt sich dabei um ungefärbte und gefärbte oder bedruckte Fasermaterialien z.B. aus Seide, Wolle, Polyamid oder Polyurethanen, und insbesondere cellulosehaltige Fasermaterialien aller Art. Solche Fasermaterialien sind beispielsweise natürliche Cellulosefasern, wie Baumwolle, Leinen, Jute und Hanf, sowie Zellstoff und regenerierte Cellulose. Bevorzugte geeignete textile Fasermaterialien sind aus Baumwolle.

Weiterhin finden die Stilbenverbindungen der Formel (1) Verwendung in Wasch- und Reinigungsformulierungen, wie z.B. in Flüssig- und Pulverwaschmitteln oder Weichspülern.

Die erfindungsgemäss eingesetzten Stilbenverbindungen eignen sich auch zur Behandlung, insbesondere zur antimikrobiellen Ausrüstung oder Konservierung von Kunststoffen, wie z.B. Polyethylen, Polypropylen, Polyurethan, Polyester, Polyamid, Polycarbonat, Latex etc.. Einsatzbereiche dafür sind z.B. Fussbodenbeläge, Kunststoffbeschichtungen, Kunststoffbehälter- und Verpackungsmaterialien; Küchen- und Badezimmer-Utensilien (z.B. Bürsten, Duschvorhänge; Schwämme, Badezimmermatten), Latex, Filtermaterialien (Luftund Wasserfilter), Kunststoffartikel, die im medizinischen Bereich eingesetzt werden, wie z.B. Verbandmaterialien, Spritzen, Katheter etc., sog. "medical devices", Handschuhe und Matratzen.

Auch Papier, wie z.B. Hygienepapiere können mit den erfindungsgemässen Stilbenverbindungen antimikrobiell ausgerüstet werden.

Weiterhin können Nonwovens, wie z.B. Windeln, Damenbinden, Dameneinlagen, Tücher für den Hygiene- und Haushaltsbereich erfindungsgemäss antimikrobiell ausgerüstet werden.

Die Stilbenverbindungen können insbesondere auch in Haushalts- und Allzweckreinigern zur Reinigung und Desinfektion von harten Oberflächen eingesetzt werden.

Ein Reinigungsmittel hat z.B. folgende Zusammensetzung:

0,01 bis 5 % der Verbindung der Formel (1)
3,0 % Octylalkohol 4EO
1,3 % Fettalkohol $C_8$-$C_{10}$-Polyglucosid
3,0 % Isopropanol
ad 100 % Wasser.

Neben der Konservierung von Kosmetik- und Haushaltsprodukten ist auch die Konservierung und antimikrobielle Ausrüstung von technischen Produkten möglich wie Papierbehandlungsflotten, Druckverdickern aus Stärke oder Celluloseabkömmlingen, Lacken und Anstrichfarben.

Die Stilbenverbindungen der Formel (1) eignen sich auch zur antimikrobiellen Holzbehandlung sowie zur antimikrobiellen Behandlung, Konservierung und Ausrüstung von Leder.

Weiterhin eignen sich die erfindungsgemässen Verbindungen zum Schutz von kosmetischen Produkten und Haushaltsprodukten vor mikrobieller Verderbnis.

Die folgenden Beispiele dienen der Veranschaulichung der Erfindung, ohne diese auf die Beispiele zu beschränken.

Beispiel 1: Herstellung von 3,5-Dihydroxystilben

1. Stufe:

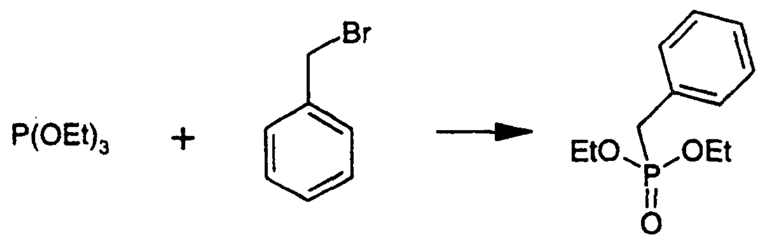

Man erhitzt die Mischung aus 51,3 g (0,3 mol) Benzylbromid und 79,1 g (0,5 mol) Triethylphosphit bis zur Beendigung der Gasentwicklung (3 h) auf 130°C. Der Überschuss an Triethylphosphit wird anschließend im Wasserstrahlvakuum abgezogen. Das Rohprodukt kann ohne weitere Reinigung für die nächste Umsetzung verwendet werden.
Ausbeute: 60 g (0,29 mol; 96,6 % d.Th.)

2. Stufe:

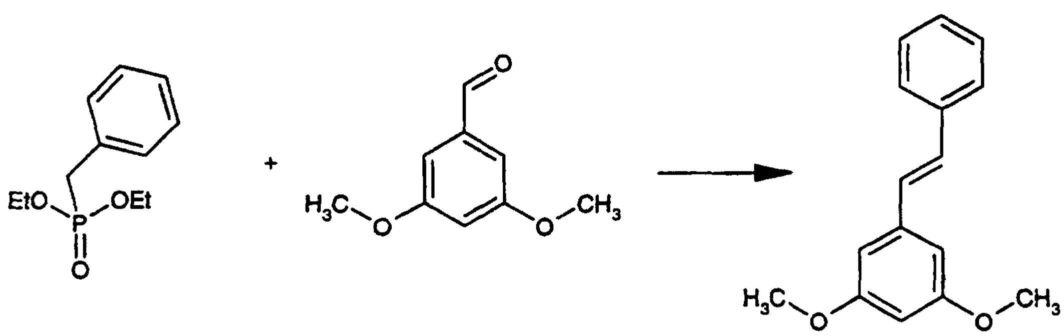

Man versetzt die Lösung von 60 g (0,29 mol) rohem Diethylbenzylphosphonat in 415 ml wasserfreiem DMF bei 0°C mit 16,5 g (0,3 mol) Natriummethylat. Anschließend werden bei 0°C insgesamt 50,0 g (0,3 mol) 3,5-Dimethoxybenzaldehyd portionsweise zugegeben. Nach 1 h Rühren bei Raumtemperatur und 1 h Erhitzen unter Rückfluß wird das Produkt durch Zugabe von 660 ml Wasser/Methanol (Mischungsverhältnis 2:1) gefällt. Die Umkristallisation aus Wasser/Methanol (2:1) liefert 3,5-Dimethoxystilben als farblose Kristalle.
Ausbeute: 54,0 g (0,22 mol, 73,3 % d.Th.)

3. Stufe:

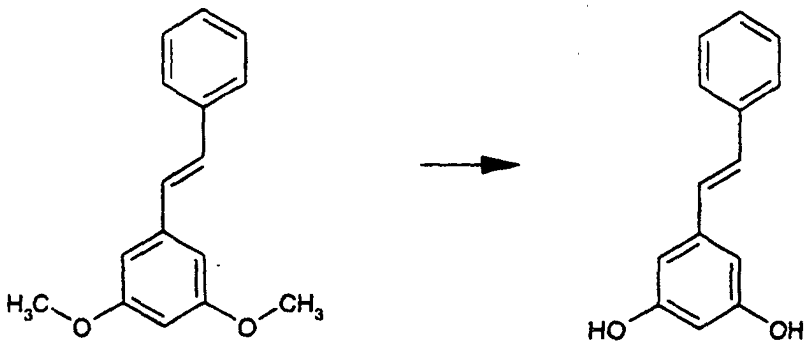

Zur Demethylierung wird die homogene Mischung von 54,0 g (0,22 mol) 3,5-Dimethoxystilben und 40,0 g (0,35 mol) Pyridinhydrochlorid 3 h auf ca. 165°C erhitzt. Anschließend trägt man die abgekühlte, ölige Reaktionsmasse in 1,2 l 2 N Salzsäure ein und isoliert das Rohprodukt durch Extraktion mit Diethylether. Die Umkristallisation aus Toluol liefert 3,5-Dihydroxystilben als schwachgelbes Pulver.
Ausbeute: 26,0 g (0,12 mol; 41,0 % d.Th.)

Beispiel 2:

Analog Beispiel 1 werden durch die Umsetzung von 20,0 g (0,12 mol) Benzylbromid, 38,9 g (0.23 mol) Triethylphosphit und 15,9 g (0.12 mol) 3-Methoxybenzaldehyd 7,0 g 3-Hydroxystilben, entsprechend der Formel

(102) 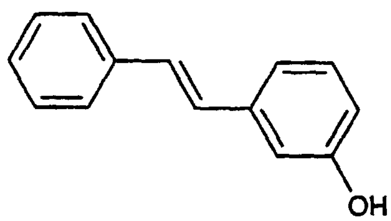

erhalten.

Beispiel 3: Bestimmung der minimalen Hemmkonzentration (MHK) im Agardiffusionstest

| | |
|---|---|
| Medium: | Mueller-Hinton Agar ( Merck)<br>* Sabouraud 4% Glucose-Agar (Merck) |
| Verdünnungsmedium: | sterile 0,85% NaCl-Lösung |
| Testkeime: | Staphylococcus aureus ATCC 9144<br>Corynebacterium xerosis ATCC 373<br>Escherichia coli NCTC 8196<br>Pseudomonas aeruginosa CIP A-22<br>Candida albicans ATCC 10231<br>* Aspergillus niger ATCC 6275 |
| Inkubation: | 24 Stunden bei 37°C<br>* 3 Tage bei 28°C |

Testlösung: Von allen Testsubstanzen werden 5%ige Stammlösungen in einem geeigneten Lösungsmittel hergestellt und in Verdünnungesreihen auf Endkonzentrationen von 1000 ppm bis 10 ppm verdünnt.

Testprinzip: 0,3 ml der jeweiligen Verdünnungsstufe werden mit 15 ml noch flüssigem Nährmedium gemischt. Nach Erstarren des Nährbodens werden von den Teststämmen punktweise je 10 µl der folgenden Keimverdünnung in 0,85 % NaCl-Lösung auf das Agarmedium aufgetragen:

| | |
|---|---|
| Staphylococcus aureus ATCC 9144 | 1:10-Verdünnung |
| Corynebacterium xerosis ATCC 373 | 1:100-Verdünnung |
| Escherichia coli NCTC 8196 | 1:100-Verdünnung |
| Pseudomonas aeruginosa CIP A-22 | 1:100-Verdünnung |
| Candida albicans ATCC 10231 | 1:10-Verdünnung |
| * Aspergillus niger ATCC 6275 | 1:10-Verdünnung |

Die Platten werden während 24 Stunden bei 37°C bebrütet (A. niger 3 Tage bei 28°C) und anschliessend die höchste Verdünnung der Testsubstanz bestimmt, bei der gerade kein Wachstum mehr beobachtet wird (entspricht MHK)

Die Ergebnisse zeigen eine starke antimikrobielle Aktivität der Testsubstanzen gegenüber gram-positiven und gram-negativen Bakterien sowie Pilzen.

Die Testergebnisse für die unten aufgeführten Verbindungen sind in Tabelle 1 aufgeführt:

Allgemeine Formel:

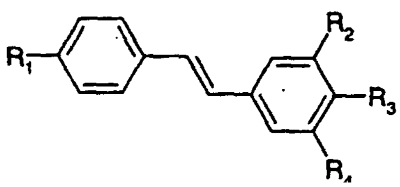

| Verbindung der Formel | $R_1$ | $R_2$ | $R_3$ | $R_4$ |
|---|---|---|---|---|
| (101) | H | OH | H | OH |
| (102) | H | OH | H | H |
| (103) | OH | OH | H | OH |
| (104) | H | H | $N(CH_3)_2$ | H |
| (105) | OH | H | H | H |
| (106) | OH | H | Cl | H |

Tabelle 1:

| Keime | Verbindung der Formel (101)[1] | Verbindung der Formel (102)[2] | Verbindung der Formel (103)[3] | Verbindung der Formel (104)[4] | Verbindung der Formel (105)[5] | Verbindung der Formel (106)[6] |
|---|---|---|---|---|---|---|
| S. aureus | 100 | 100 | 600 | 300 | - | 10 |

[1] Lösung EtOH
[2] Lösung in DMSO
[3] Lösung in EtOH
[4] Lösung in DMSO
[5] Lösung in DMSO
[6] Lösung in DMSO

Tabelle 1: (fortgesetzt)

| Keime | Verbindung der Formel (101)[1] | Verbindung der Formel (102)[2] | Verbindung der Formel (103)[3] | Verbindung der Formel (104)[4] | Verbindung der Formel (105)[5] | Verbindung der Formel (106)[6] |
|---|---|---|---|---|---|---|
| C. xerosis | -- | - | 100 | - | -- | - |
| E. coli | 100 | 100 | 600 | - | -- | -- |
| P. aeruginosa | 1000 | -- | 600 | -- | -- | -- |
| C. albicans | 100 | 100 | 600 | 600 | -- | -- |
| A. niger | 100 | 10 | -- | -- | 10 | 600 |
| (Alle Werte MHK Konzentrationen in ppm)<br>-- = nicht getestet | | | | | | |

[1] Lösung EtOH
[2] Lösung in DMSO
[3] Lösung in EtOH
[4] Lösung in DMSO
[5] Lösung in DMSO
[6] Lösung in DMSO

Beispiele 4 bis 87: Festphasensynthesemethode

Die nachfolgenden Hydroxystilbene werden nach bekannten Verfahren (R. Willard et al., Chemistry & Biology, 2,1995, 45-51) synthetisiert.
Die Reakion erfolgt nach folgendem Schema:

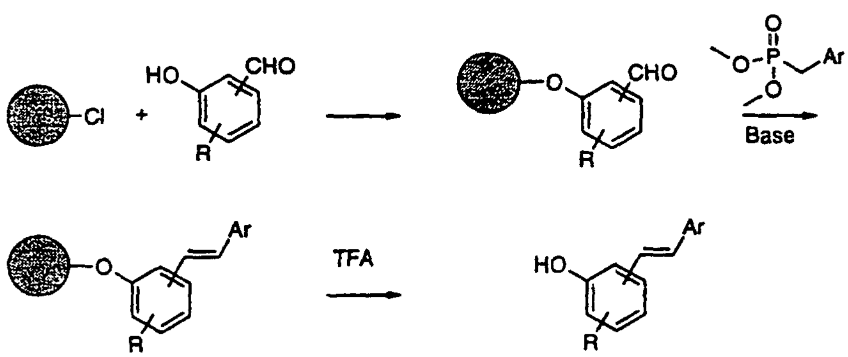


Mit dieser Methode wird eine Matrix von 12 x 7 = 84 Hydroxystilbenen mit folgendem Strukturumfang synthetisiert:

Allgemeine Formel:

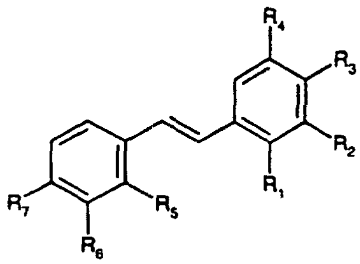

| Verbindung d. F. | $R_1$ | $R_2$ | $R_3$ | $R_4$ | $R_5$ | $R_6$ | $R_7$ |
|---|---|---|---|---|---|---|---|
| 107 | H | H | OH | H | H | H | H |
| 108 | H | OH | H | H | H | H | H |
| 109 | H | OH | OMe | H | H | H | H |
| 110 | H | OEt | OH | H | H | H | H |
| 111 | H | OMe | OH | H | H | H | H |
| 112 | OH | H | OMe | H | H | H | H |
| 113 | OH | H | H | H | H | H | H |
| 114 | H | OH | OMe | OMe | H | H | H |
| 115 | H | Me | OH | Me | H | H | H |
| 116 | OH | Me | H | H | H | H | H |
| 117 | OH | H | OBzl | H | H | H | H |
| 118 | OMe | H | OH | H | H | H | H |
| 119 | H | H | OH | H | H | H | Cl |
| 120 | H | OH | H | H | H | H | Cl |
| 121 | H | OH | OMe | H | H | H | Cl |
| 122 | H | OEt | OH | H | H | H | Cl |
| 123 | H | OMe | OH | H | H | H | Cl |
| 124 | OH | H | OMe | H | H | H | Cl |
| 125 | OH | H | H | H | H | H | Cl |
| 126 | H | OH | OMe | OMe | H | H | Cl |
| 127 | H | Me | OH | Me | H | H | Cl |

| Verbindung d. F. | $R_1$ | $R_2$ | $R_3$ | $R_4$ | $R_5$ | $R_6$ | $R_7$ |
|---|---|---|---|---|---|---|---|
| 128 | OH | Me | H | H | H | H | Cl |
| 129 | OH | H | OBzl | H | H | H | Cl |
| 130 | OMe | H | OH | H | H | H | Cl |
| 131 | H | H | OH | H | H | H | OMe |
| 132 | H | OH | H | H | H | H | OMe |
| 133 | H | OH | OMe | H | H | H | OMe |
| 134 | H | OEt | OH | H | H | H | OMe |
| 135 | H | OMe | OH | H | H | H | OMe |
| 136 | OH | H | OMe | H | H | H | OMe |
| 137 | OH | H | H | H | H | H | OMe |
| 138 | H | OH | OMe | OMe | H | H | OMe |
| 139 | H | Me | OH | Me | H | H | OMe |
| 140 | OH | Me | H | H | H | H | OMe |
| 141 | OH | H | OBzl | H | H | H | OMe |
| 142 | OMe | H | OH | H | H | H | OMe |
| 143 | H | H | OH | H | H | H | Ph |
| 145 | H | OH | H | H | H | H | Ph |
| 146 | H | OH | OMe | H | H | H | Ph |
| 147 | H | OEt | OH | H | H | H | Ph |
| 148 | H | OMe | OH | H | H | H | Ph |
| 149 | OH | H | OMe | H | H | H | Ph |
| 150 | OH | H | H | H | H | H | Ph |
| 151 | H | OH | OMe | OMe | H | H | Ph |
| 152 | H | Me | OH | Me | H | H | Ph |
| 153 | OH | Me | H | H | H | H | Ph |
| 154 | OH | H | OBzl | H | H | H | Ph |
| 155 | OMe | H | OH | H | H | H | Ph |
| 156 | H | H | OH | H | 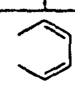 | | H |
| 157 | H | OH | H | H | 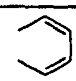 | | H |

| Verbindung d. F. | R1 | R2 | R3 | R4 | R5 | R6 | R7 |
|---|---|---|---|---|---|---|---|
| 158 | H | OH | OMe | H | 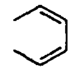 | | H |
| 159 | H | OEt | OH | H | 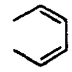 | | H |
| 160 | H | OMe | OH | H | 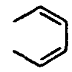 | | H |
| 161 | OH | H | OMe | H | 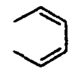 | | H |
| 162 | OH | H | H | H | 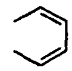 | | H |
| 163 | H | OH | OMe | OMe | 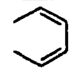 | | H |
| 164 | H | Me | OH | Me | 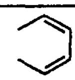 | | H |
| 165 | OH | Me | H | H | 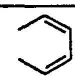 | | H |
| 166 | OH | H | OBzl | H | 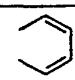 | | H |
| 167 | OMe | H | OH | H | 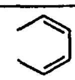 | | H |
| 168 | H | H | OH | H | Me | H | H |
| 169 | H | OH | H | H | Me | H | H |
| 170 | H | OH | OMe | H | Me | H | H |
| 171 | H | OEt | OH | H | Me | H | H |
| 172 | H | OMe | OH | H | Me | H | H |
| 173 | OH | H | OMe | H | Me | H | H |
| 174 | OH | H | H | H | Me | H | H |
| 175 | H | OH | OMe | OMe | Me | H | H |
| 176 | H | Me | OH | Me | Me | H | H |
| 177 | OH | Me | H | H | Me | H | H |
| 178 | OH | H | OBzl | H | Me | H | H |

| Verbindung d. F. | $R_1$ | $R_2$ | $R_3$ | $R_4$ | $R_5$ | $R_6$ | $R_7$ |
|---|---|---|---|---|---|---|---|
| 179 | OMe | H | OH | H | Me | H | H |
| 180 | H | H | OH | H | H | H | Me |
| 181 | H | OH | H | H | H | H | Me |
| 182 | H | OH | OMe | H | H | H | Me |
| 183 | H | OEt | OH | H | H | H | Me |
| 184 | H | OMe | OH | H | H | H | Me |
| 185 | OH | H | OMe | H | H | H | Me |
| 186 | OH | H | H | H | H | H | Me |
| 187 | H | OH | OMe | OMe | H | H | Me |
| 188 | H | Me | OH | Me | H | H | Me |
| 189 | OH | Me | H | H | H | H | Me |
| 190 | OH | H | OBzl | H | H | H | Me |
| 191 | OMe | H | OH | H | H | H | Me |

Me = Methyl
Et = Ethyl
Bzl = Benzyl

Die ermittelten mikrobiologischen Daten sind in Tab. 2 zusammengefasst.

Tabelle 2:

| MHK-Werte in ppm bei verschiedenen Mikroorganismen.*) | | | | | |
|---|---|---|---|---|---|
| Verbindung d. F. | S. heminis | E. coli | P. aeruginosa | C. albicans | A. niger |
| 107 | 100 | >100 | >100 | >100 | 100 |
| 108 | 60 | 30 | 100 | 30 | 30 |
| 109 | >100 | >100 | >100 | >100 | 100 |
| 110 | >100 | >100 | >100 | >100 | >100 |
| 111 | >100 | >100 | >100 | >100 | 100 |
| 112 | --- - | --- | --- | --- | --- |
| 113 | 100 | 100 | >100 | 60 | 60 |
| 114 | >100 | 100 | >100 | >100 | 100 |
| 115 | --- | -- | --- | --- | --- |
| 116 | 60 | 60 | 100 | 60 | 60 |
| 117 | >100 | >100 | >100 | >100 | 100 |

*) Die MHK-Werte wurden über Messung der optische Dichte bei Konzentrationen der Substanzen zwischen 100; 10 und 1 ppm ermittelt. Insofern sind einige der Daten Richtwerte der Aktivität. Die MHK Werte der Verbindungen mit guter Aktivität wurden über Messung der optischen Dichte bei Konzentrationen zwischen 120; 60; 30; 15; 7,5; 3,75 ppm ermittelt.

Tabelle 2: (fortgesetzt)

| MHK-Werte in ppm bei verschiedenen Mikroorganismen.*) | | | | | |
|---|---|---|---|---|---|
| Verbindung d. F. | S. heminis | E. coli | P. aeruginosa | C. albicans | A. niger |
| 118 | -- | --- | --- | --- | --- |
| 119 | 7,5 | >100 | >100 | 15 | 100 |
| 120 | 7,5 | 7,5 | >100 | 7,5 | 30 |
| 121 | >100 | >100 | >100 | >100 | >100 |
| 122 | >100 | >100 | >100 | >100 | >100 |
| 123 | >100 | >100 | >100 | >100 | 100 |
| 124 | 15 | 15 | >100 | 30 | 30 |
| 125 | 60 | 15 | 100 | 60 | 60 |
| 126 | >100 | >100 | >100 | 100 | 100 |
| 127 | --- | --- | --- | --- | --- |
| 128 | >100 | >100 | >100 | >100 | 100 |
| 129 | --- | --- | --- | --- | --- |
| 130 | --- | --- | --- | --- | --- |
| 131 | >100 | >100 | >100 | >100 | >100 |
| 132 | >100 | >100 | >100 | >100 | >100 |
| 133 | >100 | >100 | >100 | >100 | >100 |
| 134 | >100 | >100 | >100 | >100 | >100 |
| 135 | >100 | >100 | >100 | >100 | >100 |
| 136 | --- | --- | --- | --- | --- |
| 137 | >100 | 100 | >100 | >100 | >100 |
| 138 | >100 | >100 | >100 | >100 | >100 |
| 139 | --- | --- | --- | --- | --- |
| 140 | >100 | >100 | >100 | >100 | 100 |
| 141 | --- | --- | --- | --- | --- |
| 142 | --- | --- | --- | --- | --- |
| 143 | >100 | >100 | >100 | >100 | >100 |
| 145 | >100 | >100 | >100 | >100 | >100 |
| 146 | >100 | >100 | >100 | >100 | >100 |
| 147 | >100 | >100 | >100 | >100 | >100 |
| 148 | >100 | >100 | >100 | >100 | >100 |
| 149 | --- | --- | --- | --- | --- |
| 150 | >100 | >100 | >100 | >100 | >100 |
| 151 | >100 | >100 | >100 | >100 | >100 |
| 152 | --- | --- | --- | --- | --- |

*) Die MHK-Werte wurden über Messung der optische Dichte bei Konzentrationen der Substanzen zwischen 100; 10 und 1 ppm ermittelt. Insofern sind einige der Daten Richtwerte der Aktivität. Die MHK Werte der Verbindungen mit guter Aktivität wurden über Messung der optischen Dichte bei Konzentrationen zwischen 120; 60; 30; 15; 7,5; 3,75 ppm ermittelt.

Tabelle 2: (fortgesetzt)

| MHK-Werte in ppm bei verschiedenen Mikroorganismen.*) | | | | | |
|---|---|---|---|---|---|
| Verbindung d. F. | S. heminis | E. coli | P. aeruginosa | C. albicans | A. niger |
| 153 | >100 | >100 | >100 | >100 | >100 |
| 154 | --- | --- | --- | --- | --- |
| 155 | --- | --- | --- | --- | --- |
| 156 | 7,5 | 60 | >100 | 15 | 30 |
| 157 | 7,5 | 15 | >100 | 15 | 60 |
| 158 | >100 | >100 | >100 | >100 | >100 |
| 159 | >100 | >100 | >100 | >100 | 100 |
| 160 | >100 | >100 | >100 | >100 | 100 |
| 161 | --- | --- | --- | --- | --- |
| 162 | 7,5 | 30 | >100 | 30 | 15 |
| 163 | >100 | >100 | >100 | >100 | 100 |
| 164 | --- | --- | --- | --- | --- |
| 165 | 100 | >100 | >100 | >100 | 100 |
| 166 | --- | --- | --- | --- | --- |
| 167 | --- | -- | --- | --- | --- |
| 168 | 7,5 | 30 | 100 | 15 | 30 |
| 169 | 30 | 60 | 100 | 30 | 30 |
| 170 | >100 | >100 | >100 | >100 | >100 |
| 171 | >100 | >100 | >100 | >100 | >100 |
| 172 | >100 | >100 | >100 | 10 | >100 |
| 173 | -- | --- | --- | --- | --- |
| 174 | 60 | 100 | >100 | 60 | 60 |
| 175 | 60 | 60 | 100 | 60 | 100 |
| 176 | -- | --- | --- | --- | --- |
| 177 | 30 | 60 | >100 | 60 | 60 |
| 178 | --- | --- | --- | --- | --- |
| 179 | --- | --- | --- | --- | --- |
| 180 | 100 | >100 | >100 | >100 | >100 |
| 181 | 15 | 15 | 100 | 15 | 30 |
| 182 | >100 | >100 | >100 | >100 | >100 |
| 183 | >100 | >100 | >100 | >100 | >100 |
| 184 | >100 | >100 | >100 | >100 | 100 |
| 185 | --- | --- | --- | --- | --- |
| 186 | 30 | 30 | 100 | 30 | 60 |

*) Die MHK-Werte wurden über Messung der optische Dichte bei Konzentrationen der Substanzen zwischen 100; 10 und 1 ppm ermittelt. Insofern sind einige der Daten Richtwerte der Aktivität. Die MHK Werte der Verbindungen mit guter Aktivität wurden über Messung der optischen Dichte bei Konzentrationen zwischen 120; 60; 30; 15; 7,5; 3,75 ppm ermittelt.

Tabelle 2: (fortgesetzt)

| MHK-Werte in ppm bei verschiedenen Mikroorganismen.*) | | | | | |
|---|---|---|---|---|---|
| Verbindung d. F. | S. heminis | E. coli | P. aeruginosa | C. albicans | A. niger |
| 187 | >100 | >100 | >100 | >100 | >100 |
| 188 | --- | --- | --- | --- | --- |
| 189 | >100 | 100 | >100 | >100 | >100 |
| 190 | -- | --- | --- | --- | --- |
| 191 | --- | --- | --- | --- | --- |
| --- = nicht bestimmt | | | | | |

*) Die MHK-Werte wurden über Messung der optische Dichte bei Konzentrationen der Substanzen zwischen 100; 10 und 1 ppm ermittelt. Insofern sind einige der Daten Richtwerte der Aktivität. Die MHK Werte der Verbindungen mit guter Aktivität wurden über Messung der optischen Dichte bei Konzentrationen zwischen 120; 60; 30; 15; 7,5; 3,75 ppm ermittelt.

Bestimmung der minimalen Hemmkonzentration (MHK-Wert) in Mikrotiterplatten:

Nährmedium:

Casein-Sojamehl-Pepton-Bouillon zur Herstellung der Vorkulturen der Testbakterien und Hefe.
Mycological Schrägagar zur Vorkultur von Schimmelpilzen

Beispiele für Testkeime:

Bakterien: Staphyluococcus hominis DMS 20328 Escherichia coli NCTC 8196 Pseudomonas aeruginosa CIP A-22

Hefe: Candida albicans ATCC 10231 Schimmelpilz: Aspergillus niger ATCC 6275

Durchführung:

Die Testsubstanzen werden in Dimethylsulfoxid 8DMSO) vorgelöst und in einer Verdünnungsreihe von 1:2 getestet.
Bakterien und Hefe werden über Nacht in CASO-Bouillon, der Schimmelpilz auf Mycological-Schrägagar angezüchtet und mit 10 ml 0,85 % Kochsalzlösung (+ 0, 1 % TritonX-100) abgeschwämmt.
Alle Testkeime werden mit 0,85 %iger Kochsalzlösung auf eine Keimzahl von $1{,}5X10^6$ KBE/ml eingestellt.
Die Testsubstanzen werden à 8 µl pro well in Mikrotiterplatten vorpipettiert.
Vorverdünnte Keimsubstanzen werden 1:100 in CASO-Bouillon (Bakterien und Hefe) bzw. Sabouraud 2% Glucsoe-Bouillon (Schimmelpilz) verdünnt und à 192 µl pro well den Testsubstanzen zugegeben.
Die Testansätze werden 48 Stunden bei 37°C (Bakterien und Hefe) bzw. 5 Tage bei 28°C (Schimmelpilz) inkubiert.
Nach Inkubation wird das Wachstum anhand der Trübung der Testansätze (otische Dichte) bei 620 nm in einem Mikroplate-Reader bestimmt.
Als minimale Hemmkonzentration (MHK-Wert) wird diejenige Substanzkonzentration angegeben, bei der (verglichen mit der Wachstumskontrolle) eine deutliche Wachstumshemmung (≤ 20 % Wachstum) der Testkeime festzustellen ist.
Pro Testkeim und Substanzkonzentration wird eine Mikrotiterplatte angesetzt. Alle Substanzen werden im Doppel geprüft.

**Patentansprüche**

1. Nichttherapeutische Verwendung von Hydroxystilbenverbindungen der Formel

(1) 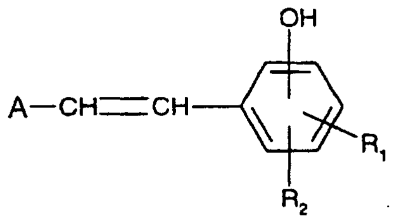 ,

worin

A einen Rest der Formel (1a)

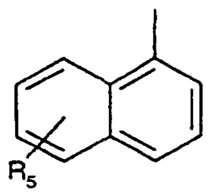 ;

und

$R_1$, $R_2$ und $R_5$, unabhängig voneinander Wasserstoff, Halogen, Hydroxy, $C_1$-$C_{16}$-Alkyl, $C_1$-$C_{16}$-Alkoxy, Phenyl; $C_1$-$C_3$-Phenylalkyl; $C_6$-$C_{10}$-Aryloxy, Amino, Mono-$C_1$-$C_5$-Alkylamino, Di-$C_1$-$C_5$-Alkylamino, oder -$NO_2$;

bedeuten, zur Abtötung und/oder Hemmung des Wachstums von pathogenen grampositiven und gramnegativen Bakterien sowie Bakterien der Hautflora, Hefen und Schimmelpilzen, **dadurch gekennzeichnet, dass** man eine Oberfläche ausgewählt aus der Haut, der Schleimhäute und der Haare mit einer Verbindung der Formel (1) behandelt.

**2.** Verwendung nach Anspruch 1, **dadurch gekennzeichnet, dass** in den Formeln (1)
$R_1$ und $R_2$ Hydroxy
bedeuten.

**3.** Verwendung nach Anspruch 1 oder 2, **dadurch gekennzeichnet, dass** die Verbindungen der Formel (1) in der E- oder Z-Form vorliegen.

**4.** Verwendung nach Anspruch 3, **dadurch gekennzeichnet, dass** die Verbindungen der Formel (1) in der E-Form vorliegen.

**5.** Verwendung nach Anspruch 1, **dadurch gekennzeichnet, dass** Verbindungen der Formel

(3) 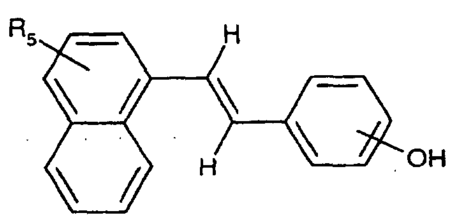

verwendet werden, worin
$R_5$ die in Formel (1) angegebene Bedeutung hat.

**6.** Verwendung der Verbindung der Formel

(1) 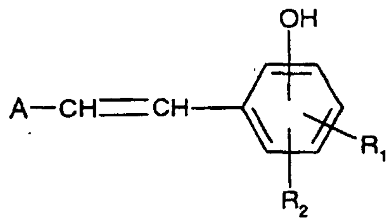 ,

worin

A einen Rest der Formel (1a)

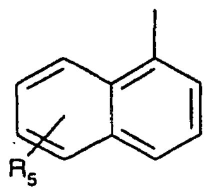 ;

und

$R_1$, $R_2$ und $R_5$, unabhängig voneinander Wasserstoff, Halogen, Hydroxy, $C_1$-$C_{16}$-Alkyl, $C_1$-$C_{16}$-Alkoxy, Phenyl; $C_1$-$C_3$-Phenylalkyl; $C_6$-$C_{10}$-Aryloxy, Amino, Mono-$C_1$-$C_5$-Alkylamino, Di-$C_1$-$C_5$-Alkylamino, oder -$NO_2$;

bedeuten,
in Wasch- und Reinigungsformulierungen.

**7.** Verwendung der Verbindung der Formel

(1) 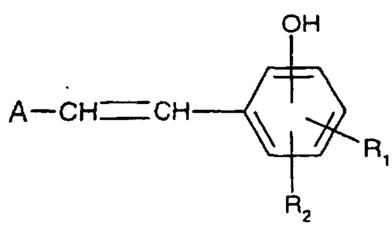 ,

worin

A einen Rest der Formel (1a)
und

$R_1$, $R_2$ und $R_5$, unabhängig voneinander Wasserstoff, Halogen, Hydroxy, $C_1$-$C_{16}$-Alkyl, $C_1$-$C_{16}$-Alkoxy, Phenyl; $C_1$-$C_3$-Phenylalkyl; $C_6$-$C_{10}$-Aryloxy, Amino, Mono-$C_1$-$C_5$-Alkylamino, Di-$C_1$-$C_5$-Alkylamino, oder -$NO_2$:

bedeuten,
zur antimikrobiellen Ausrüstung und Konservierung von Kunststoffen, Papier, Nonwovens, Holz oder Leder.

## Claims

**1.** Nontherapeutic use of hydroxystilbene compounds of formula

(1) 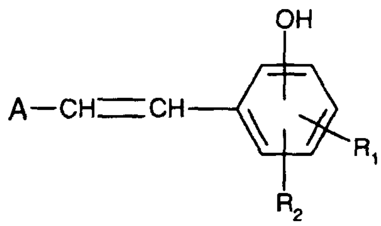 ,

wherein

A is a radical of formula (1a)

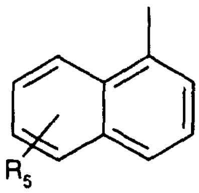 ;

and

$R_1$, $R_2$ and $R_5$ are each independently of the others hydrogen, halogen, hydroxy, $C_1$-$C_{16}$alkyl, $C_1$-$C_{16}$alkoxy, phenyl; $C_1$-$C_3$phenylalkyl; $C_6$-$C_{10}$aryloxy, amino, mono-$C_1$-$C_5$alkylamino, di-$C_1$-$C_5$alkylamino, or -$NO_2$;

for the aborticide and and/or the inhibition of pathogenic gram positive and gram negative bacteria as well as bacteria of the skin flora, yeasts and moulds which comprises treating the surface selected from the skin, mucosa and hair with a compound of formula (1).

**2.** Use according to claim 1, wherein, in formulae (1), $R_1$ and $R_2$ are hydroxy.

**3.** Use according to claim 1 or 2, wherein the compounds of formula (1) are present in the E-or Z-form.

**4.** Use according to claim 3, wherein the compounds of formula (1) are present in the E-form.

**5.** Use according to claim 1, wherein there are used compounds of formula

(3) 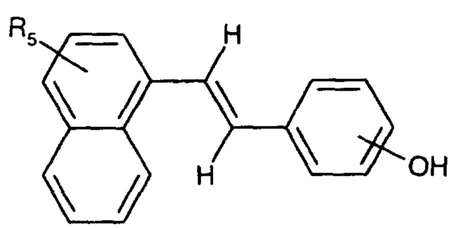

wherein
$R_5$ is as defined for formula (1).

**6.** Use of the compound of formula

(1) 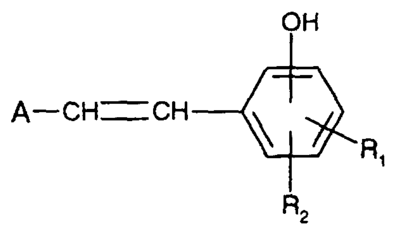 ,

wherein

A is a radical of formula (1a)

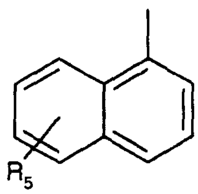 ;

and

$R_1$, $R_2$ and $R_5$ are each independently of the others hydrogen, halogen, hydroxy, $C_1$-$C_{16}$alkyl, $C_1$-$C_{16}$alkoxy, phenyl; $C_1$-$C_3$phenylalkyl; $C_6$-$C_{10}$aryloxy, amino, mono-$C_1$-$C_5$alkylamino, di-$C_1$-$C_5$alkylamino, or $-NO_2$;

in washing and cleaning formulations.

**7.** Use of the compound of formula

(1) 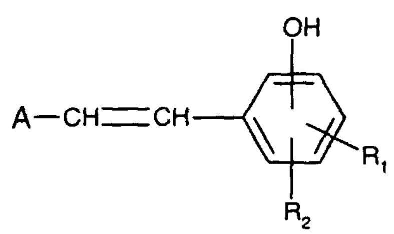 ,

wherein

A is a radical of formula (1a)

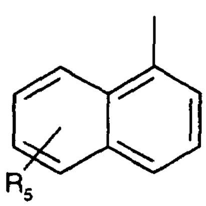 ;

and

$R_1$, $R_2$ and $R_5$ are each independently of the others hydrogen, halogen, hydroxy, $C_1$-$C_{16}$alkyl, $C_1$-$C_{16}$alkoxy, phenyl; $C_1$-$C_3$phenylalkyl; $C_6$-$C_{10}$aryloxy, amino, mono-$C_1$-$C_5$alkylamino, di-$C_1$-$C_5$alkylamino, or -$NO_2$;

in imparting antimicrobial properties to and preserving plastics, paper, nonwovens, wood or leather.

**Revendications**

**1.** Utilisation non thérapeutique de composés d'hydroxystilbène de formule

(1) 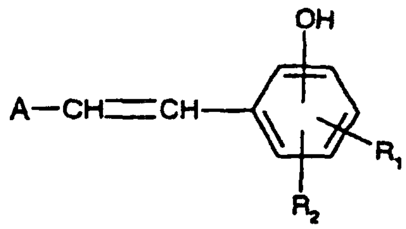 ,

dans laquelle

A représente un groupe de formule (Ia)

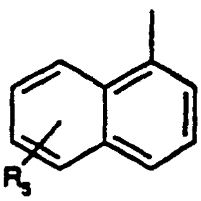 ;

et

$R_1$, $R_2$ et $R_5$ représentent, indépendamment les uns des autres, un atome d'hydrogène, un atome d'halogène, un groupe hydroxy, alkyle en $C_1$ à $C_{16}$, alcoxy en $C_1$ à $C_{16}$, phényle ; (phényle en $C_1$ à $C_3$)alkyle ; (aryle en $C_6$ à $C_{10}$)oxy, amino, mono(alkyle en $C_1$ à $C_5$)amino, di(alkyle en $C_1$ à $C_5$)amino ou -$NO_2$ ;

pour éliminer et/ou inhiber la croissance de bactéries pathogènes Gram-positives et Gram-négatives et de bactéries de la flore cutanée, de levures et de moisissures, **caractérisée en ce que** l'on traite une surface, choisie parmi la peau, les muqueuses et les cheveux, avec un composé de formule (1).

**2.** Utilisation selon la revendication 1, **caractérisée en ce que** $R_1$ et $R_2$ dans les formules (1) représentent un groupe hydroxy.

**3.** Utilisation selon la revendication 1 ou 2, **caractérisée en ce que** les composés de formule (1) sont présents sous forme cis ou trans.

**4.** Utilisation selon la revendication 3, **caractérisée en ce que** les composés de formule (1) sont présents sous forme trans.

**5.** Utilisation selon la revendication 1, **caractérisée en ce que** l'on utilise des composés de formule

(3) 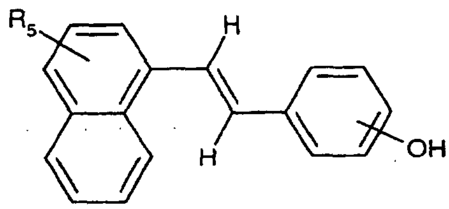

dans laquelle

$R_5$ a la signification indiquée dans la formule (1).

**6.** Utilisation du composé de formule

(1) 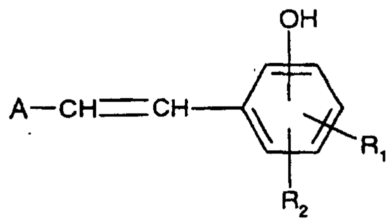 ,

dans laquelle

A représente un groupe de formule (1a)

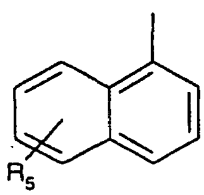 ;

et

$R_1$, $R_2$ et $R_5$ représentent, indépendamment les uns des autres, un atome d'hydrogène, un atome d'halogène, un groupe hydroxy, alkyle en $C_1$ à $C_{16}$, alcoxy en $C_1$ à $C_{16}$, phényle ; (phényle en $C_1$ à $C_3$)alkyle ; (aryle en $C_6$ à $C_{10}$)oxy, amino, mono(alkyle en $C_1$ à $C_5$)amino, di(alkyle en $C_1$ à $C_5$)amino ou $-NO_2$ ;

dans des formulations lavantes et nettoyantes.

**7.** Utilisation du composé de formule

(1) 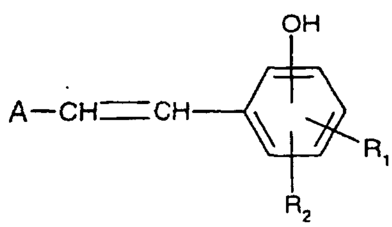 ,

dans laquelle

A représente un groupe de formule (Ia)

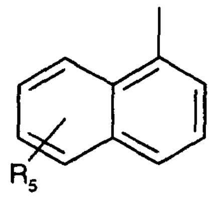

;

et

$R_1$, $R_2$ et $R_5$ représentent, indépendamment les uns des autres, un atome d'hydrogène, un atome d'halogène, un groupe hydroxy, alkyle en $C_1$ à $C_{16}$, alcoxy en $C_1$ à $C_{16}$, phényle ; (phényle en $C_1$ à $C_3$)alkyle ; (aryle en $C_6$ à $C_{10}$)oxy, amino, mono (alkyle en $C_1$ à $C_5$)amino, di(alkyle en $C_1$ à $C_5$)amino ou $-NO_2$ ;

pour le traitement antimicrobien et la conservation de matières plastiques, du papier, de non tissés, du bois ou du cuir.